# EUROPEAN PATENT APPLICATION

(11) **EP 4 501 356 A1**
(43) Date of publication of application: **05.02.2025**
(21) Application number: 23775068.2
(22) Date of filing: 24.03.2023
(51) Int. Cl.: A61K 45/00, A23L 33/10, A23L 33/17, A61K 31/19, A61K 31/198, A61K 31/7016, A61P 43/00

(54) **SENOLYSIS BY AMMONIA-LOWERING COMPONENT**

(30) Priority: 25.03.2022 JP 2022049514
(71) Applicant: Taz Inc., Tokyo 113-0033 (JP)
(72) Inventor: KAMIMOTO TAKAHASHI, Shoko, Tokyo 113-0033 (JP); SAITO, Kenji, Tokyo 113-0033 (JP)
(74) Representative: Plasseraud IP
(86) International application number: PCT/JP2023/011704
(87) International publication number: WO 2023/182475

(57) **Abstract**

An object of the present invention is to develop a means for removing senescent cells present in a living body with simpler method with fewer side effects. The inventors of the present invention focused on a mechanism of maintaining the survival of senescent cells by ammonia which is generated in excess through metabolism and demonstrated that the promotion of the ammonia excretion enables the removal of senescent cells. More specifically, they revealed that when a component having an action of excreting ammonia is administered to aged mice in animal experiments using mice, the expression level of p21, a marker of senescent cells, significantly decreases. They demonstrated, for the first time, that an effect of removing the senescent-cell can be exerted by the intake of a component for removing ammonia.

## Description

### Technical Field

The present invention relates to provide a novel means for removing senescent cells from a living body and preventing aging. More specifically, the present invention relates to provide a novel means for removing senescent cells from a living body and for preventing aging by a substance having an action for promoting ammonia excretion in the body.

### Background Art

In human bodies, it is known that "senescent cells", that is, aged cells that were no longer able to proliferate, accumulate in the bodies and cause diseases, such as arteriosclerosis and fatty liver, and aging phenomena. Recent studies have revealed that when mice whose senescent cells can be removed were produced, the onset of diseases with aging was delayed in the mice (Non Patent Literature 1, Non Patent Literature 2).

It has been reported that removal of senescent cells (senolysis) can prevent or delay functional impairment of a body and can extend a healthy lifespan (Non Patent Literature 1).

However, many of the candidate medicinal agents for removing senescent cells that have been reported so far are used as anticancer drugs and there have been a concern that their side effects are too strong to be used for removing senescent cells.

### Citation List

### Non Patent Literature

Non Patent Literature 1: Johmura Y., SCIENCE, Vol 371, Issue 6526, pp. 265-270, 2021
Non Patent Literature 2: Suda M., et al., Nature Aging, vol 1, pages 1117-1126, 2021

### Summary of Invention

### Technical Problem

An object of the present invention is to develop a means for removing senescent cells in a living body and preventing aging with simpler method with fewer side effects.

### Solution to Problem

The inventors of the present invention focused on a mechanism of maintaining the survival of senescent cells by ammonia which is generated in excess through metabolism and hypothesized that the promotion of ammonia excretion in the body enables the removal of senescent cell or the prevention of aging. Then they studied that medicinal agents and food components known to have an action of promoting ammonia excretion are effective for removing senescent cell or preventing aging by using aged mice.

The inventors of the present invention focused on a mechanism of maintaining the survival of senescent cells by ammonia which is generated in excess through metabolism and demonstrated that the promotion of ammonia excretion enables the removal of senescent cell or the prevention of aging. More specifically, they revealed that when a component having an action of excreting ammonia is administered to aged mice in animal experiments using mice, the expression level of p21, a marker for senescent cells, significantly decreases. They demonstrated, for the first time, that an effect of removing a senescent-cell can be exerted by the intake of a component for removing ammonia.

More specifically, this application provides the following embodiment for solving the above objects.
[1] An agent for removing senescent-cell comprising a substance having an action for promoting ammonia excretion in the body.
[2] The agent for removing senescent-cell according to [1], wherein the substance having an action for promoting ammonia excretion in the body is one or more substances selected from the group consisting of ornithine, citrulline, taurine, carnitine, arginine, branched-chain amino acids (BCAAs), acetyl glutamic acid, sodium benzoate, lactulose, lactitol, sodium phenylbutyrate, sodium phenylacetate, rifaximin, and carglumic acid.
[3] The agent for removing senescent-cell according to [1], wherein the substance having an action for promoting ammonia excretion in the body is lactulose, ornithine, sodium benzoate, or any combination thereof.
[4] An agent for preventing aging comprising a substance having an action for promoting ammonia excretion in the body.
[5] The agent for preventing aging according to [4], wherein the substance having an action for promoting ammonia excretion in the body is one or more substances selected from the group consisting of ornithine, citrulline, taurine, carnitine, arginine, branched-chain amino acids (BCAAs), acetyl glutamic acid, sodium benzoate, lactulose, lactitol, sodium phenylbutyrate, sodium phenylacetate, rifaximin, and carglumic acid.
[6] The agent for preventing aging according to [4], wherein the substance having an action for promoting ammonia excretion in the body is lactulose, ornithine, sodium benzoate, or any combination thereof.
[7] A composition having an action for removing senescent-cell or an action for preventing aging, the composition comprising a substance having an action for promoting ammonia excretion in the body.
[8] The composition according to [7] for medical use.
[9] The composition according to [7] or [8], wherein the substance having an action for promoting ammonia excretion in the body is one or more substances selected from the group consisting of ornithine, citrulline, taurine, carnitine, arginine, branched-chain amino acids (BCAAs), acetyl glutamic acid, sodium benzoate, lactulose, lactitol, sodium phenylbutyrate, sodium phenylacetate, rifaximin, and carglumic acid.
[10] The composition according to [7] or [8], in which the substance having an action for promoting ammonia excretion in the body is lactulose, ornithine, sodium benzoate, or any combination thereof.

### Advantageous Effects of Invention

According to the present invention, it is possible to provide an agent for removing senescent-cell or an agent for preventing aging, and a composition having an action for removing senescent-cell or an action for preventing aging comprising a substance having an action for promoting ammonia excretion in the body. By the agent for removing senescent-cell, the agent for preventing aging, or a composition having an action for removing senescent-cell or an action for preventing aging, it is possible to provide a means for removing senescent cells in a living body and preventing aging with simpler method just by oral intake, without side effects associated with anticancer drugs.

### Brief Description of Drawings

[Figure 1] Figure 1 shows the results of measuring mRNA expression level of p21, a marker of senescent cells, in the kidney.
[Figure 2] Figure 2 shows the results of measuring mRNA expression level of p21 and p16, markers of senescent cells, in the liver.
[Figure 3] Figure 3 shows the results of measuring mRNA expression level of p16, a marker of senescent cells, in the hippocampus.
[Figure 4] Figure 4 shows the results of measuring mRNA expression level of p16, a marker of senescent cells in the skeletal muscle.

### Description of Embodiments

The present inventors paid attention to a mechanism of maintaining the survival of senescent cells by ammonia metabolically generated excessively and demonstrated that senescent cells can be removed by promoting the excretion of ammonia.

As a result, according to a first aspect of the present invention, it is possible to provide an agent for removing senescent-cell comprising a substance having an action for promoting ammonia excretion in the body.

Generally, a senescent cell refers to a cell that has irreversibly terminated cell division due to chromosomal DNA damage, which is caused by various stresses such as telomere abnormality, oxidative stress, DNA damage, activation of a cancer genes, mitochondrial dysfunction, viral infection, and inflammation. It is known that p16 gene and p21 gene are highly expressed in senescent cells and considered as representative markers for senescent cells. Concerning function, it is known that accumulation of senescent cells in a body leads to high expression of various inflammatory proteins such as cytokines and chemokines and their extracellular secretion, causing, e.g., functional deterioration, chronic inflammation and carcinogenesis of various tissues.

Based on these features, it has been proposed that aging and geriatric diseases can be ameliorated by removing senescent cells by senolytic drugs (Non Patent Literature 1).

Common features of senescent cells are conceivably as follows: the lysosomal membrane of a cell is damaged with accumulation of defective proteins and the content of the inner lumen of lysosomes, which have a pH about pH5, leaks into the interior of the cell, with the result that intracellular pH decreases; production of a glutamine-metabolizing enzyme, i.e., glutaminase 1 (GLS1), is activated in such a cell, resulting in the excessive production of glutamic acid and ammonia from glutamine; and the ammonia produced excessively can control the homeostasis of the intracellular pH, resulting in maintaining survival of the cells.

Thus, the inventors of the present invention conceived that the intracellular pH of senescent cells is acidified by removing ammonia from the interior of the cells such that senescent cells cannot survive, resulting in promotion of the removal of senescent cells.

There are several pathways for excreting ammonia from the interior of a cell. More specifically, the ammonia excretion pathways are known as follows:
- Urea cycle (ornithine cycle) in which ornithine or citrulline reacts with ammonia to finally produce urea, which is then detoxified and excreted,
- Production of an acid amide in which glutamic acid (Glu) or aspartic acid (Asp) reacts with ammonia to produce glutamine (Gln) or asparagine (Asn),
- Reaction with a keto acid, in which glutamic acid (Glu) is produced by the reaction between alpha-ketoglutarate and ammonia,
- Production of creatine,
- Pathway of reducing blood ammonia concentration by activating the urea cycle,
- Pathway of reducing blood ammonia by acting on intestinal bacteria to decrease ammonia production, and
- Pathway of excreting as hippuric acid through biding to ammonia.

A substance having an action for promoting ammonia excretion in the body that can be used as an active ingredient in the present invention refers to a substance that can act on one or more ammonia excretion pathways as mentioned above and promote the excretion of ammonia. For example, the substances reacting with ammonia such as ornithine or citrulline, which are constituents of the ornithine cycle; or glutamic acid or aspartic acid in production of an acid amide; and alpha-ketoglutarate involved in the reaction with keto acid, can be used for this purpose, but the substances are not limited to these materials.

More specifically, the substance include, but are not limited to, for example, ornithine, citrulline, taurine, carnitine, arginine, branched-chain amino acids (BCAAs), acetyl glutamic acid, sodium benzoate, lactulose, lactitol, sodium phenylbutyrate, sodium phenylacetate, rifaximin, and carglumic acid. For example, any substance that is used for treating hyperammonemia currently under clinical study, can be used in future for the purpose of the present invention.

According to a preferable embodiment of the present invention, lactulose, ornithine, sodium benzoate, or any combination thereof can be used as the above-mentioned substance having an action for promoting ammonia excretion in the body,.

The agent for removing senescent-cell of the present invention may comprise not only the aforementioned active ingredient but also various additives commonly used in foods or medicines.

In the present invention, the case of removing senescent cells may include a case of removing senescent cells from a living body by inducing cell-death of the senescent cells, and a case of changing the properties of senescent cells to reduce the characterization of the senescent cells. Whether the senescent cells were removed or not can be confirmed using samples collected from a living body based on whether the expression levels of p16 gene and p21 gene, characteristic genes of the senescent cells, decreased or not; whether the activity of aging-associated acidic beta-galactosidase (SA beta-Gal) decreased or not; and the degree of cell staining (SA beta-Gal staining, HE staining, PAS staining, Sirius red staining, Masson's trichrome staining).

In the present invention, the amount of intake of the active ingredient varies depending on the substance to be used as an active ingredient and may be determined as the amount of intake generally used and specified depending on the substance. The intake schedule for each of the active ingredients may be any schedule, for example, once a day, three times a day, or less frequency such as once a week or once a month.

As a derivative of the first embodiment of the present invention, the present invention can also provide an agent for preventing aging comprising a substance having an action for promoting ammonia excretion in the body. In the present invention, as mentioned above, removing senescent cells by a substance having an action for promoting ammonia excretion in the body as the active ingredient enables to prevent aging.

According to the second embodiment of the present invention, the present invention can provide a composition having an action for removing senescent-cell or an action for preventing aging, which comprises a substance having an action for promoting ammonia excretion in the body and.

As previously mentioned, the substance having an action for promoting ammonia excretion in the body that can be used as the active ingredient in the present invention may include substances present in a living body, substances contained in foods and substances that are needed to have approval as medicines. For this reason, the composition in the embodiment may be a food composition (a composition commonly used in food and a composition of food having a bioregulatory function such as food for specified health uses, food with nutritional functions, or food with functional claims) or a pharmaceutical composition.

The composition of the present invention may comprise not only the aforementioned active ingredient (a substance having an action for promoting ammonia excretion in the body) but also other ingredients and various types of additives commonly used in foods or medicines, which are acceptable regardless of their types of ingredients and additives.

When the composition having an action for removing senescent-cell or an action for preventing aging which comprises a substance having an action for promoting ammonia excretion in the body is used as a pharmaceutical composition, the composition is usually administrated via the oral route. However, the administration method can be changed depending on the site where the target senescent cells to be removed are present and the composition can be administered via routes other than the oral route.

For example, for removing senescent cell or preventing aging in a tissue accessible via blood, the active ingredient of the present invention can be administered via the oral administration, or intravenous injection, subcutaneous injection, direct administration into the tissue, and intraperitoneal injection.

For example, for removing senescent cell or preventing aging in the brain which is difficult to access directly via blood, the ingredients of the present invention can be administered using administration route such as epidural administration, intraventricular administration, or nasal administration.

The present invention will be described in more detail by way of Examples. However, the present invention is not limited by Examples in any manner.

### Example

### Example 1: Study on effect of removing senescent-cell

In this Example, various types of substances having an effect of promoting an ammonia excretion were administered to animals to confirm whether or not the substances are effective for removing senescent cells.

### (1) Sample preparation

As animals, experiments was performed using 7 young mice (male C57BU6J mice, 7 weeks old) and 28 aged mice (male C57BU6J-Aged mice, 78 weeks old).

The animals were kept in an SPF environment with a temperature of 20 to 26°C a humidity of 30 to 70%, and 12-hours lighting/day (turned on at 7:00 and turned off at 19:00). The temperature and humidity were recorded every day and were stored as raw data.

Animals were raised by individual housing in polycarbonate mouse cages (170 W × 240 D × 120 H mm, manufactured by CLEA Japan, Inc.) as breeding cages. As the bedding, paper chips and Paper-clean (manufactured by Japan SLC, Inc.) sterilized by an autoclave (temperature 121°C, sterilized for 40 minutes, dried for 30 minutes) were used.

As feedstuff, solid feed for mouse/rat MF (manufactured by Oriental Yeast Co., Ltd.) sterilized by autoclave (at temperature 121°C, sterilized for 15 minutes, dried for 5 minutes) was allowed to take ad libitum.

As water, water containing 0.025% of sodium hypochlorite (Turukuron, manufactured by Tsurumi Soda Co., Ltd.) was put in a self-pumped 100 mL-polycarbonate water bottle and allowed to drink ad libitum.

Group 1 was constituted of young mice, and aged mice were divided into 4 groups each consisting of 7 mice (Groups 2 to 5) and the experimental conditions of individual groups are set as shown in the Table below.

**Table 1**

| Group | Name of Group | Drinking Water | Number of Mice (Identification Number) | Mice |
|---|---|---|---|---|
| 1 | Young | Normal water | 7 (M1-M7) | Young mice |
| 2 | Aged | Normal water | 7 (M8-M14) | Aged mice |
| 3 | Aged + O | Component A | 7 (M15-M21) | Aged mice |
| 4 | Aged + OB | Component B | 7 (M22-M28) | Aged mice |
| 5 | Aged + LO | Component C | 7( M29-M35) | Aged mice |

As explained below, water comprising a test substance was given to the mice of Group 3 to Group 5. The test substances to be given to the mice of Group 3 to Group 5 were Component A, Component B, and Component C, respectively, and prepared in the following manner twice a week.
- Component A: L-ornithine hydrochloride (99%, Sigma-Aldrich) was added to water so as to have a concentration of 1%.
- Component B: L-ornithine hydrochloride (99%, Sigma-Aldrich) was added to water so as to have a concentration of 1% and sodium benzoate (> 99%, Sigma-Aldrich) was added to the water so as to have a concentration of 0.0006%.
- Component C: LACTULOSE syrup (60%, Kowa Pharmaceutical Co., Ltd.) was added to water so as to have a concentration of 7.2%.

The each types of drinking water which were prepared by adding a test substance as mentioned above was given to mice of each of the groups ad libitum, and the test substance was orally administered for one month. One month after the initiation of the experiment, all mice were sacrificed and dissected. From each of the mice, the liver, kidney, hippocampus and skeletal muscle were collected for measurement markers for senescent cells.

### (2) Measurement of marker for senescent cells

### (2-1) RNA extract

The tissues were collected from mice at the time of dissection and soaked in RNAlater (Ambion) overnight and thereafter stored at -80°C. Total RNA was extracted from the liver tissue by the TRIzol-chroloform method.

The tissue extracted from RNAlater (50 to 100 mg) was put in 1 ml of TRIzol Reagent (Invitrogen Life Technologies), crushed. After standing still at room temperature for 5 minutes, 0.2 ml of chloroform was added and stirred. After standing still at room temperature for 2 to 3 minutes, the mixture was centrifuged at 4°C and 12,000 × g for 15 minutes. The upper layer (250 micro l) was recovered and 0.5 ml of isopropanol was added. After standing still at room temperature for 10 minutes, the mixture again centrifuged at 4°C and 12,000 × g for 10 minutes.

The RNA was purified with 75% ethanol, evaporated to dry under reduced pressure, and dissolved in 50 micro l of RNA-free water. Using NanoDrop ND-1000 (NanoDrop Technologies), absorbance was measured at 260 nm and 280 nm to determination of an absorbance ratio (A260/280 nm), RNA purity was confirmed by checking whether or not the absorbance ratio falls within the range of 1.9 to 2.1.

### (2-2) Real-time RT-PCR

Quantitative real-time PCR was performed using total RNA extracted from the tissue for analysis of mRNA expressions of senescence markers p16 and p21 commonly known in the technical field.

From the total RNA, cDNA was synthesized using PrimeScript (Registered trademark) RT reagent Kit (Perfect Real Time) in accordance with the protocol of the manufacturer. Then, cDNA was amplified by Thermal Cycler Dice Real Time System TP800 (Takara Bio) and fluorescence was detected by SYBR (Registered trademark) Premix Ex Taq^{™} (Perfect Real Time) and real-time PCR detection system (Takara Bio).

The PCR reaction was carried out by performing a single cycle at 95°C × 10 sec, then 40 cycles of [95°C × 5 sec, 60°C × 30 sec] and further a single cycle of [95°C × 15 sec, 60°C × 30 sec, 95°C × 15 sec].

Using the mRNA expression of glyceraldehyde-3-phosphate dehydrogenase (GAPDH) as an internal standard for correction, the relative values of mRNAs of interest were calculated. Each primer was designed by web application PRIMER3 and was synthesized by outsourcing to Invitrogen. Note that, the sequences of the primers for amplifying target genes of senescence markers p16 and p21 are as shown below.

### [Formula 1]

p16 forward primer: CGTACCCCGATTCAGGTGAT (SEQ ID NO: 1)
p16 reverse primer: TTGAGCAGAAGAGCTGCTACGT (SEQ ID NO: 2)
p21 forward primer: CCTGGTGATGTCCGACCTG (SEQ ID NO: 3)
p21 reverse primer: CCATGAGCGCATCGCAATC (SEQ ID NO: 4)

### (2-3) Effect of removing senescent-cell

In the tissue samples of the liver, kidney, hippocampus and skeletal muscle collected in the above (1), the expressions of senescence markers p16 and p21 were confirmed by PCR amplification, as mentioned in (2-1) and (2-2). The reference symbols "*", "**", and "***" shown indicate p < 0.05, p < 0.01, and p < 0.005, respectively in the following Figures.

### (2-3-1) Expression level in the kidney

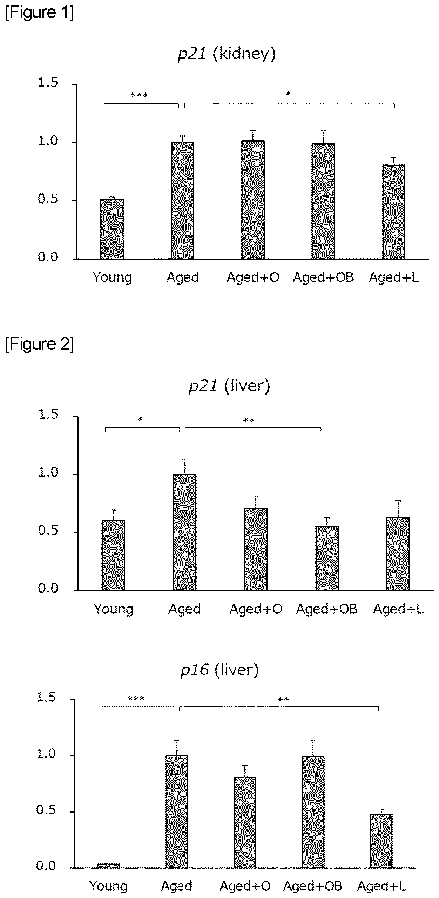

When the mRNA expression level of marker p21 for senescent cells was measured in the kidney, the expression level significantly decreased in the group of aged mice to which lactulose was given (in the Figure, indicated as "Aged + L"), compared to that of the aged mice having no additional administration (in the Figure, indicated as "Aged") (Figure 1).

### (2-3-2) Expression level in the liver

When, in the liver, the mRNA expression level of marker p21 for senescent cells was measured, the mRNA expression levels of p21 significantly decreased in all experimental groups (ornithine intake group ("Aged + O"), ornithine + sodium benzoate group ("Aged + OB"), lactulose intake group ("Aged + L")), compared to that of the aged mice having no additional administration (in the Figure, indicated as "Aged") (Figure 2, upper panel). In particular, in the ornithine + sodium benzoate intake group (in the Figure, indicated as "Aged + OB"), the expression level significantly decreased, and the expression level was tended to decrease in the ornithine intake group (in the Figure, indicated as "Aged + O") and the lactulose intake group (in the Figure, indicated as "Aged + L") (p < 0.1).

When, in the liver, the mRNA expression level of marker p16 for senescent cells was also measured, the mRNA expression level of p16 was tended to decrease in the ornithine intake group (in the Figure, indicated as "Aged + O"), compared to that in the aged mice (in the Figure, indicated as "Aged") having no additional administration. In the lactulose intake group (in the Figure, indicated as "Aged + L"), the mRNA expression level of p16 significantly decreased (Figure 2, lower panel).

### (2-3-3) Expression level in the hippocampus

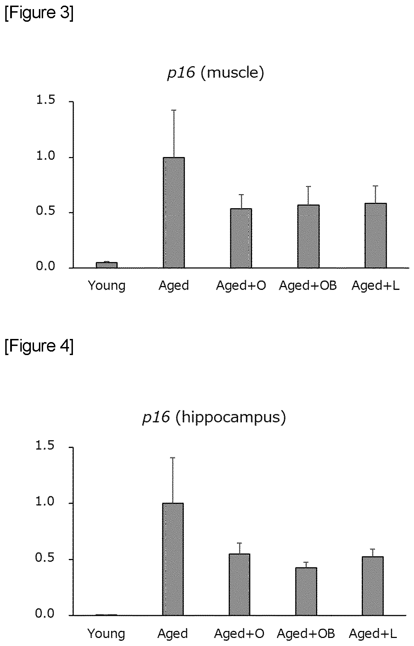

When, in the hippocampus, the mRNA expression level of marker p16 for senescent cells was measured, the mRNA expression level of p16 decreased, although not significantly, in all experimental groups (ornithine intake group (in the Figure, indicated as "Aged + O"), ornithine + sodium benzoate intake group (in the Figure, indicated as "Aged + OB"), and lactulose intake group (in the Figure, indicated as "Aged + L")), compared to the group of aged mice (in the Figure, indicated as "Aged") having no additional administration (Figure 3).

### (2-3-4) Expression level in the skeletal muscle

When, in the skeletal muscle, the mRNA expression level of marker p16 for senescent cells was measured, the mRNA expression level of p16 decreased, although not significantly, in all experimental groups (ornithine intake group (in the Figure, indicated as "Aged + O"), ornithine + sodium benzoate intake group (in the Figure, indicated as "Aged + OB"), and lactulose intake group (in the Figure, indicated as "Aged + L")), compared to the group of aged mice (in the Figure, indicated as "Aged") having no additional administration (Figure 4).

### Industrial Applicability

The present invention can provide an agent for removing senescent-cell or a composition having an action for removing senescent-cell comprising a substance having an action for promoting ammonia excretion in the body. By the agent for removing senescent-cell or the composition having an action for removing senescent-cell, it is possible to provide a means for removing senescent cells in the living body with simpler method just by oral intake, without side effects associated with anticancer drugs.

Sequence Listing Information:
   DTD Version: V1_3
   File Name: Removal of senescent cells. xml
   Software Name: WIPO Sequence
   Software Version: 2.1.2
   Production Date: 2023-03-23
General Information:
   Current application / IP Office: JP
   Current application / Applicant file reference: 088P001WO
   Earliest priority application / IP Office: JP
   Earliest priority application / Application number: JP2022-49514
   Earliest priority application / Filing date: 2022-03-25
   Applicant name (designation): Fantagene Inc.
   Applicant name (designation) / Language: ja
   Applicant name (designation) / Name (Latin alphabet): Fantagene Inc.
   Inventor name: Shoko Takahashi
   Inventor name / Language: ja
   Inventor name / Name (Latin alphabet): Shoko Takahashi
   Invention title: Removal of senescent cells by ammonia-lowering components (en)
   Invention title: Removal of senescent cells by ammonia-lowering components (ja)
   Sequence Total Quantity: 4
Sequences:
   Sequence Number (ID): 1
   Length: 20
   Molecular Type: DNA
   Features Location/Qualifiers:
      - misc_feature, 1..20
         > note, Forward primer for p16 gene
      - source, 1..20
         > mol_type, other DNA
         > organism, synthetic construct
   Residues:
      cgtaccccga ttcaggtgat
      20
   Sequence Number (ID): 2
   Length: 22
   Molecular Type: DNA
   Features Location/Qualifiers:
      - misc_feature, 1..22
         > note, Reverse primer for p16 gene
      - source, 1..22
         > mol_type, other DNA
         > organism, synthetic construct
   Residues:
      ttgagcagaa gagctgctac gt 22
   Sequence Number (ID): 3
   Length: 19
   Molecular Type: DNA
   Features Location/Qualifiers:
      - misc_feature, 1..19
         > note, Forward primer for p21 gene
      - source, 1..19
         > mol_type, other DNA
         > organism, synthetic construct
   Residues:
      cctggtgatg tccgacctg
      19
   Sequence Number (ID): 4
   Length: 19
   Molecular Type: DNA
   Features Location/Qualifiers:
      - misc_feature, 1..19
         > note, Reverse primer for p21 gene
      - source, 1..19
         > mol_type, other DNA
         > organism, synthetic construct
   Residues:
      ccatgagcgc atcgcaatc 19
END

## Claims

1. An agent for removing senescent-cell comprising a substance having an action for promoting ammonia excretion in the body.

2. The agent for removing senescent-cell according to claim 1, wherein the substance having an action for promoting ammonia excretion in the body is one or more substances selected from the group consisting of ornithine, citrulline, taurine, carnitine, arginine, branched-chain amino acids (BCAAs), acetyl glutamic acid, sodium benzoate, lactulose, lactitol, sodium phenylbutyrate, sodium phenylacetate, rifaximin, and carglumic acid.

3. The agent for removing senescent-cell according to claim 1, wherein the substance having an action for promoting ammonia excretion in the body is lactulose, ornithine, sodium benzoate, or any combination thereof.

4. An agent for preventing aging comprising a substance having an action for promoting ammonia excretion in the body.

5. The agent for preventing aging according to claim 4, wherein the substance having an action for promoting ammonia excretion in the body is one or more substances selected from the group consisting of ornithine, citrulline, taurine, carnitine, arginine, branched-chain amino acids (BCAAs), acetyl glutamic acid, sodium benzoate, lactulose, lactitol, sodium phenylbutyrate, sodium phenylacetate, rifaximin, and carglumic acid.

6. The agent for preventing aging according to claim 4, wherein the substance having an action for promoting ammonia excretion in the body is lactulose, ornithine, sodium benzoate, or any combination thereof.

7. A composition having an action for removing senescent-cell or an action for preventing aging, the composition comprising a substance having an action for promoting ammonia excretion in the body.

8. The composition according to claim 7 for medical use.

9. The composition according to claim 7 or 8, wherein the substance having an action for promoting ammonia excretion in the body is one or more substances selected from the group consisting of ornithine, citrulline, taurine, carnitine, arginine, branched-chain amino acids (BCAAs), acetyl glutamic acid, sodium benzoate, lactulose, lactitol, sodium phenylbutyrate, sodium phenylacetate, rifaximin, and carglumic acid.

10. The composition according to claim 7 or 8, wherein the substance having an action for promoting ammonia excretion in the body is lactulose, ornithine, sodium benzoate, or any combination thereof.
